# EUROPEAN PATENT APPLICATION

(11) **EP 1 923 461 A1**
(43) Date of publication of application: **21.05.2008**
(21) Application number: 07254450.5
(22) Date of filing: 14.11.2007
(51) Int. Cl.: C12M 1/04, B01F 13/02

(54) **A bioreactor**

(30) Priority: 15.11.2006 US 859077 P; 15.11.2006 US 859178 P; 16.11.2006 US 859445 P
(71) Applicant: MILLIPORE CORPORATION, Billerica, Massachusetts 01821-3405 (US)
(72) Inventor: Belongia, Brett, Andover Massachusetts 01845 (US); Schauer, Neil, Milford Massachusetts 01757 (US)
(74) Representative: Greenwood, John David

(57) **Abstract**

A bioreactor formed of a material such as glass or plastic is provided having a first closed wall spaced apart from a second closed wall. At least a portion of the height of the first closed wall extends toward the center of the bioreactor so that gas introduced at the bioreactor bottom contacts the first closed wall prior to contacting the top surface of reactants in the bioreactor.

## Description

### BACKGROUND OF THE INVENTION

The culture of microbial cells (fermentation) or animal and plant cells (tissue culture) are commercially-important chemical and biochemical production processes. Living cells are employed in these processes because living cells, using generally easily obtainable starting materials, can economically synthesize commercially-valuable chemicals including proteins such as monoclonal antibodies or enzymes; vaccines or alcoholic beverages. In order to establish optimum commercial culture process conditions, it is common practice to effect bioreaction in small scale culture shaker flasks or roller bottles wherein test reaction conditions can be precisely controlled.

Fermentation involves the growth or maintenance of living cells in a nutrient liquid media. In a typical batch fermentation process, the desired microorganism or eukaryotic cell is placed in a defined medium composed of water, nutrient chemicals and dissolved gases, and allowed to grow (or multiply) to a desired culture density. The liquid medium must contain all the chemicals which the cells require for their life processes and also should provide the optimal environmental conditions for their continued growth and/or replication. Currently, a representative microbial cell culture process might utilize either a continuous stirred-tank reactor or a gas-fluidized bed reactor in which the microbe population is suspended in circulating nutrient media. Similarly, in vitro mammalian cell culture might employ a suspended culture of cells in roller flasks or, for cells requiring surface attachment, cultures grown to confluence in tissue culture flasks containing nutrient medium above the attached cells. The living cells, so maintained, then metabolically produce the desired product(s) from precursor chemicals introduced into the nutrient mixture. The desired product(s) are either purified from the liquid medium or are extracted from the cells themselves.

One system for a bioreactor has been to use a large table, equipped with motors or hydraulics onto which a bioreactor bag is placed. The motors/hydraulics rock the bag providing constant movement of the cells. Additionally, the bag has a gas and nutrient supply tube and a waste gas and waste product tube which allow for the supply of nutrients and gases such as air for aerobic organisms and the removal of waste such as respired gases, carbon dioxide and the like. The tubes are arranged to work with the motion of the bag to allow for a uniform movement of the gases and fluids/solids. See U.S. Pat. No. 6,190,913. Such a system requires the use of capital-intensive equipment, with components that are susceptible to wear. Additionally, the size of the bag that can be used with the table is limited by the size of table and the lifting capability of its motors/hydraulics. Also, the apparatus for rocking the bag comprises a heat source which renders it more difficult to control reaction temperatures in the bag.

An alternative system uses a long flexible tube-like bag that has both ends attached to movable arms such that the bag after filling is suspended downwardly from the movable arm in the shape of a U. The arms are then alternately moved upward or downward relative to the other so as to cause a rocking motion and fluid movement within the bag. If desired, the midsection may contain a restriction to cause a more intimate mixing action. This system requires the use of a specifically shaped bag and hydraulic or other lifting equipment to cause the movement of the liquid. Additionally, due to weight considerations, the bag size and volume is restricted by the lifting capacity of the equipment and the strength of the bag.

An improvement has been shown through the use of one or more bags that are capable of being selectively pressurized and deflated in conjunction with a disposable bio bag such as a fermenter, mixing bag, storage bag and the like. The pressure bag(s) may surround a selected outer portion of the bag or may be contained within an inner portion of such a bag. By selectively pressurizing and deflating the pressure bag(s), one is able to achieve fluid motion in the bag thereby ensuring cell suspension, mixing and/or gas and/or nutrient/excrement transfer within the bag without damaging shear forces or foam generation.

Alternatively, one can select a static (non-moving) bag that contains a sparger or other device for introducing a gas into the bag. The gas causes the movement of the fluid in the bag as well to cause the mixing and transfer of gases, nutrients and waste products.

U.S. Pat. No. 5,565,015 uses a flat, inflatable porous tube that is sealed into a plastic container. The tube inflates under gas pressure and allows gas to flow into the bag. When the gas is not applied, the tube collapses and substantially closes off the pores of the flat tube to prevent leakage from the bag.

U.S. Pat. No. 6,432,698 also inserts and seals a tube to a gas diffuser within the bag. It appears that a constant positive gas pressure must be maintained in order to prevent any liquid within the bag from entering the diffuser and then the gas line and eventually the air pump as no valve or other means from preventing backflow is shown.

Both of the structures disclosed by these two patents have the potential for leakage of the liquid in the container which can potentially contaminate the contents of the bag of the upstream components of the system such as the gas supply system. Additionally, both introduce a separate component for the gas distribution.

Accordingly, it would be desirable to provide a small scale bioreactor apparatus wherein process conditions such as temperature and degree of mixing can be easily controlled.

The present invention is as claimed in the claims.

### SUMMARY OF THE INVENTION

The present invention provides a disposable bioreactor which is shaped to affect movement of reactant liquid upwardly along an inner surface of an outer wall of the bioreactor and then downwardly within the reactant volume remote for the inner surface of the outer wall of the bioreactor.

Embodiments of the invention include a disposable bioreactor for small scale cell culture wherein optimum bioreaction process conditions can be established and wherein reaction temperature and reactant mixing can be easily controlled.

The bioreactor includes a first inner surface of an outer wall which forms a closed volume with a second inner surface of an inner wall of the bioreactor. The first and second inner surfaces each have at least a portion thereof which converge toward the centre of the bioreactor in the bottom to top direction so that movement of reactant liquid within the bioreactor is in an essentially spiral direction under the influence of gases introduced into the bioreactor.

The bioreactor includes means for introducing gas and for removing gas. The bioreactor also includes means for adding reactants and for removing desired product(s).

The bioreactor is formed of a material such as glass or a polymeric composition which does not contaminate the reactants or the products.

### BRIEF DESCRIPTION OF THE DRAWINGS

Embodiments of the invention will now be described, by way of example only, with reference to the accompanying drawings, of which:
Figure 1 is a diagrammatic side view of the bioreactor of an embodiment of the present invention;
Figure 2 is a diagrammatic cross sectional view of the bioreactor of Figure 1;
Figure 3 is a diagrammatic side view of a bioreactor of this invention which shows liquid reactant movement therein;
Figure 4 is a diagrammatic side view of a further embodiment of the present invention; and
Figure 5 is a diagrammatic cross sectional view of the embodiment of Figure 4.

### DESCRIPTION OF SPECIFIC EMBODIMENTS

In an embodiment in accordance with this invention, a disposable, bioreactor is provided having a closed inner wall and a closed outer wall which are spaced apart from each other to form a volume wherein bioreaction is affected. At least a portion of inner wall and a portion of the outer wall converge. The degree of convergence is such as to cause the reactants to move in a spiral direction within the bioreactor. A cell culture, nutrients and one or more gases are introduced into the bioreactor to effect a bioreaction therein. By the term "effective volume" as used herein is meant the bioreactor volume wherein reaction occurs. A portion of the bioreactor volume comprises a gas containing volume positioned above the effective volume. The gas is introduced into the bioreactor in a manner so that it causes mixing of the reactants and so that it contacts the inner surface of the outer wall prior to breaking the reactant upper surface. By causing the gas to move in this manner, the reactant mixture moves in an essentially spiral path over the entire circumference of the bioreactor.

The internal volume of the bioreactor is shaped so that the reactants are satisfactorily mixed together in all portions of the effective volume. Thus, dead zones where little or no mixing occurs are avoided. A volume external the bioreactor is provided to house a heater which controls temperature within the bioreactor. One or more inlets to the bioreactor are provided for the purpose of introducing reactants into the bioreactor or to remove products from the bioreactor or to remove gases from the bioreactor.

Gas is introduced into the effective volume of the bioreactor by a porous passage which can be formed integrally with the bioreactor such as by being adhered thereto along the circumference of the bioreactor. Alternatively, the porous passages can be formed separately from the bioreactor such as a sparger tube. The porous passage can be formed of a flexible material such as a polymeric composition which does not contaminate the reactants or product(s) or a rigid material such as a ceramic, a glass such as glass mat or a sintered glass material or sintered stainless steel which does not contaminate the reactants or product(s).

Suitable plastics can be hydrophilic or hydrophobic. When hydrophilic however one must ensure that the air pressure within the passage is either constantly at or above that of the liquid intrusion pressure so as to keep the liquid out of the passage or to provide an upstream shutoff such as a valve or hydrophobic filter to prevent the liquid in the bioreactor from flooding the passage and /or upstream gas supply. Plastics can be inherently hydrophilic or hydrophobic or can be surface treated to provide the desired properties. The plastics may be a single layer or if desired, multilayered. One example of a multilayered passage has a porous plastic layer covered by a more open prefilter or depth filter that can trap any debris and keep the debris from clogging the porous passage(s). The pore size or sizes selected depends upon the size of gas bubble desired. The pore size may range from microporous (0.1 to 10 microns) to macroporous (greater than 10 microns) and it may be formed of membranes or filters such as a microporous filter, woven fabrics or filters, porous non-woven materials, such as Tyvek® sheet materials, monoliths or pads, such as can be found in many aquarium filters and the like. The selected plastic(s) should be compatible with the bioreactor environment so it doesn't adversely affect the cells being grown within it. Suitable plastics include but are not limited to polyolefins such as polyethylene or polypropylene, polysulfones such as polysulfone or polyethersulfone, nylons, PTFE resin, PEF, PVDF, PET and the like.

The introduced gas functions both as a reactant and as a means for mixing the reactants.

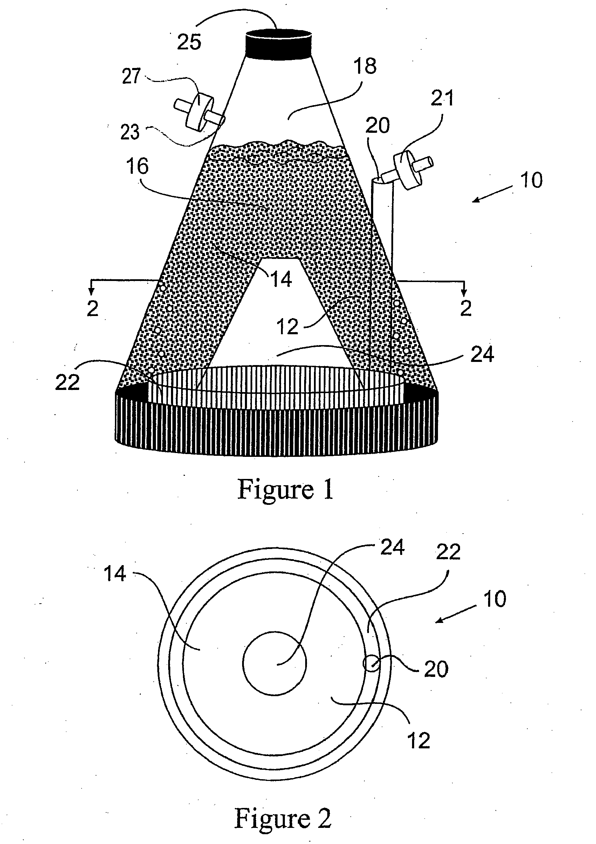

Referring to Figures 1 and 2, a bioreactor 10 includes two legs 12 and 14 which are connected by section 16 positioned above the legs 12 and 14. A gas volume 18 is provided above section 16 where unreacted introduced gas is collected. The gas is introduced into bioreactor 10 through inlet 20 having filter 21 and porous passage 22 which extends about the lower circumference of bioreactor 10. The inlet 20 is connected to a gas source (not shown). The external volume 24 is shaped to house a heater (not shown) for controlling the temperature within the bioreactor 10. A gas outlet 23 with filter 27 is also provided. A septum 25 is provided to introduce non- gaseous reactants.

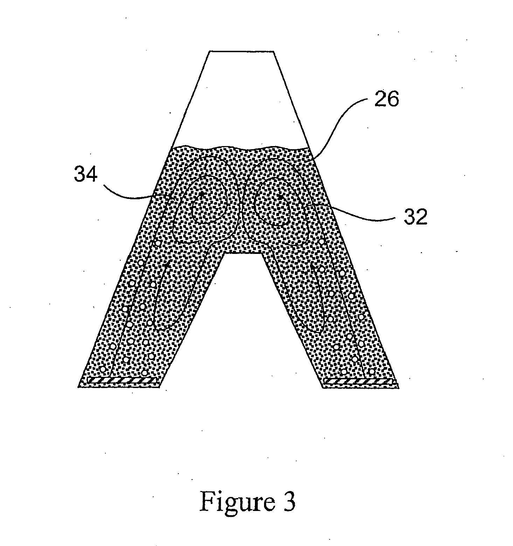

As shown in Figure 3, the cross section of the internal volume 26 is constant about the circumference of the bioreactor 10. The height of the effective volume can be maintained essentially constant by the volume of nutrients added and the volume of products removed. Thus, mixing conditions, as represented by arrows 32 and 34, are essentially constant throughout the circumference of the bioreactor 10 even after effective volume increase.

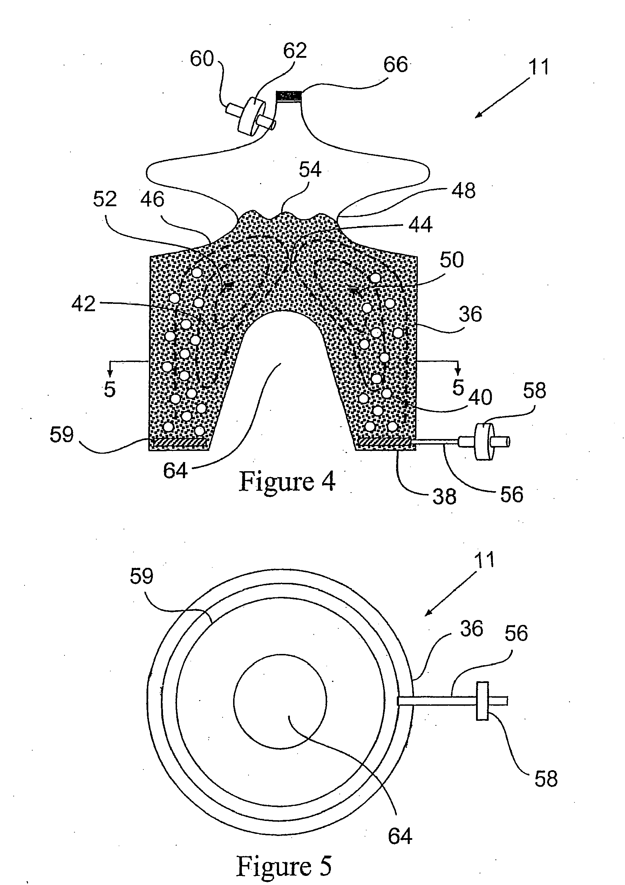

Referring to Figures 4 and 5, an alternative configuration of a bioreactor 11 of this invention is shown. The bioreactor 11 is constructed so that the outer wall 36 is positioned perpendicular to bottom surface 38 to form legs 40 and 42 which are joined by section 44. The surface 46 converges toward liquid/gas interface surface 48 so that the introduced gas moves in direction of arrows 50 and 52 prior to the gas breaking the reactant surface 54. Gas is introduced through inlet 56 having filter 58 into porous passage 59. Gas is removed through outlet 62 having filter 60. The external volume 64 is provided to house a heater (not shown). A septum 66 is provided to introduce reactants into the bioreactor 11.

A bioreactor of this invention can be formed of a rigid plastic or glass. The plastics are preferably thermoplastics and include but are not limited, polyolefins homopolymers such as polyethylene and polypropylene, polyolefins copolymers, polycarbonates, polystyrenes and the like. It is preferred that the plastic be transparent so the activity within can be monitored by visual inspection.

## Claims

1. A bioreactor having a bottom and a top and comprising a first closed wall defining a outer circumference of the bioreactor and forming an outer surface of the bioreactor, a second closed wall spaced apart from said first closed wall and defining an inner circumference of the bioreactor, means for introducing gas into the bioreactor, means for removing gas from the bioreactor, at least a portion of the height of said first closed wall converging toward the center of said bioreactor in the bottom to top direction so that gas introduced into said bioreactor contacts the portion of said first closed wall prior to contacting a top surface of reactants in the bioreactor.

2. The bioreactor of claim 1 wherein the entire first closed wall converges toward the centre.

3. The bioreactor of claim 1 wherein at least a portion of inner wall and at least a portion of the outer wall each converge towards the centre of the bioreactor in the bottom to top direction.

4. The bioreactor of claim 3 wherein the degree of convergence is such as to cause the reactants to move on a spiral path within the bioreactor.

5. The bioreactor of claim 1 wherein a cross-section of the internal volume is constant about the circumference of the bioreactor.

6. The bioreactor of claim 5 wherein the height of the effective volume can be maintained essentially constant by controlling the volume of nutrients added and the volume of products removed.

7. The bioreactor of claim 5 wherein the mixing conditions are essentially constant throughout the portion of the bioreactor even after effective volume increase.

8. The bioreactor of any preceding claim wherein the means for introducing gas is a porous passage formed of one or more layers of porous material.

9. The bioreactor of any preceding claim including an external volume adjacent an outer surface of said second closed wall.
